# EUROPÄISCHE PATENTANMELDUNG

(11) **EP 2 108 677 A1**
(43) Veröffentlichungstag der Anmeldung: **14.10.2009**
(21) Anmeldenummer: 08103488.6
(22) Anmeldetag: 10.04.2008
(51) Int. Cl.: C08L 3/02, A61K 9/48

(54) **Thermoplastische Stärkemassen**

(71) Anmelder: Swiss Caps Rechte und Lizenzen AG, 9533 Kirchberg (CH)
(72) Erfinder: Schwab, Ernst, Dr., 9500 Wil (CH); Halter, Martin, Dr., 9240 Uzwil (CH); Brocker, Erich, Dr., 9533 Kirchberg (CH)
(74) Vertreter: Hepp, Dieter

(57) **Zusammenfassung**

Die vorliegende Erfindung betrifft eine homogene, ungetrocknete, schmelzextrudierte thermoplastische Masse, enthaltend 30-60 Gew.-% Trockensubstanz an nativer oder chemisch modifizierter Stärke, höchstens 11 Gew.-% Trockensubstanz mindestens eines weiteren Biopolymers ausgewählt aus der Gruppe bestehend aus Carrageenan oder einem anderen Polysaccharid oder einem Protein, mindestens einen Weichmacher und maximal 20 Gew.-% zugesetztem Wasser. Aus dieser Masse können vorteilhafte Formkörper wie Weichkapseln mit erhöhter Schlagzähigkeit hergestellt werden.

## Beschreibung

Die vorliegende Erfindung betrifft eine Verbesserung von wasserlöslichen, stärkehaltigen Materialien für die Herstellung von Bändern mittels Schmelzextrusion, sowie die Verwendung dieser Bänder/Filme als Hüllenmaterial zur Herstellung von Formkörpern mittels dem Rotary Die-Verfahren.

Die vorliegende Erfindung betrifft Formkörper, insbesondere Kapseln, die als Darreichungsform für ernährungsphysiologisch oder pharmakologisch wirksame, aktive Stoffe - primär für die orale, rektale, vaginale Applikation - dienen. Die erfindungsgemässen Formkörper können aber auch dosierte Stoffe für technische Anwendungen, wie Lösemittel, Schmiermittel, Detergenzien, Kosmetika, Farbpasten etc. enthalten. Vorzugsweise betrifft die vorliegende Erfindung Weichkapseln aus zwei untrennbar verschweissten Hüllenteilen.

Weichkapseln können nur mit wenigen Herstellverfahren realisiert werden:
a) Coazervation,
b) Tropfverfahren (Koextrusion ohne mechanisches Formgebungsverfahren),
c) Vorformung der Hülle und anschliessend Befüllung und Versiegelung.

Weichkapseln im Sinne der vorliegenden Erfindung weisen eine Hülle aus einem flexiblem Film auf und sind nach einem Verfahren gemäss c) hergestellt. Bevorzugt besteht dieser flexible Film aus thermoplastischem Material, welches weiter bevorzugt auch zusätzlich noch wasserlöslich ist.

Zur Erhöhung der mechanischen Flexibilität kann das Material zusätzlich einen Weichmacher enthalten, also einen niedermolekularen Stoff mit niedrigem Dampfdruck, d.h. ein "Lösemittel", das permanent im Material verbleibt. Als spezieller Weichmacher ist für wasserlösliche Polymere Wasser selbst zu bezeichnen. In der Regel ist Wasser ein guter Weichmacher für wasserlösliche Polymere, hat aber eine relative hohe Flüchtigkeit (hoher Dampfdruck, relativ geringe Verdampfungsenthalpie). Da Wasserdampf praktisch ständig in der Atmosphäre und damit in der Umgebungsluft des Formkörpers vorhanden ist, ist Wasser ein Weichmacher zumindest für das wasserlösliche Polymer des Hüllenmaterials, da es durch Sorption vom Hüllenmaterial aufgenommen wird. Darüber hinaus ist Wasser für alle mindestens teilweise wasserlöslichen Polymere zumindest ein Lösemittel/Weichmacher temporärer Art: Es lassen sich daraus Lösungen (Solvent im Überschuss) des Polymers in Wasser oder "umgekehrte Lösungen" (Polymer im Überschuss, also weichgemachte Polymere) herstellen. Im Gegensatz zu permanenten Weichmachern ist aber der temporäre Weichmacher Wasser durch Trocknung zu entfernen bzw. durch Sorption wieder einzuführen. Die mechanischen Eigenschaften solcher Materialien sind damit abhängig von der Summe von permanentem und temporärem Weichmacher.

Das Herstellen von Formkörpern mit Hilfe des Rotary-Die-Verfahrens ist die wirtschaftlichste und am breitesten einsetzbare Methode zur Realisierung verschiedener Formen, Grössen und Füllgüter, wobei die Formkörper in der Regel einen Durchmesser von 2 bis 20 mm aufweisen. Bei diesem Verfahren wird die Hülle durch Verschweissen von zwei Filmen, entsprechend zweier Hüllenhälften, um einen flüssigen, oder festen Kern herum gebildet. Das Verfahren ist also grundsätzlich verschieden von Tropfverfahren, bei denen der Kern und die Hülle gleichzeitig durch gepulste, cozentrische Coextrusion von 2 nicht mischbaren Phasen gebildet werden.

Die Konstruktion der für das Rotary-Die-Verfahren erforderlichen Maschine verlangt für die Verarbeitung der Filme gewisse minimale Eigenschaften des Hüllenwerkstoffes, d.h. des kalten und heissen Films. Für den kalten Film z.B. E-Modul, Schlagzähigkeit; für den heissen Film - also unmittelbar vor bzw, während des Vorganges der Verformung und Verschweissung - ausreichende Zugspannung, Bruchdehnung, Verschweissbarkeit (Melt flow index) usw. Die Verschweissbarkeit wird duch eine ausreichende Erhöhung des melt flow index durch inkrementale Erhöhung der Temperature an der Schweissstelle und erhöhten Druck durch die nahtbildenden Werkzeuge (lap seal) erreicht. Es sollte eine ausreichend hohe Zugspannung bei möglichst geringen Klebrigkeiten bis sehr kurz vor des "Schmelz"- bzw. Verschweisstemperaturbereiches vorhanden sein. Dies wird am besten mit thermoreversiblen Gelen aus wasserlöslichen Biopolymeren sichergestellt. Die Rotary Die-Technik wurde deshalb ursprünglich für wässrige Gelatineschmelzen (Sol/Gel-Übergang bei ca. 44°C) entwickelt. Gelatinefreie Materialien basierend auf dem gleichen Prinzip, d.h. dem Sol/Gel-Übergang, wurden aus wässrigen Carrageenan- oder Gellan-Filmen realisiert. Es handelt sich hierbei um thermoreversible Gele mit einem Sol/Gel-Übergang bei ca. 60-75°C. Solche Filme enthalten ca. 35 bis 95% Wasser, abhängig davon ob nur der Gelbildner oder auch ein das sonst allzu flüssige Sol verdickendes Füllmaterial wie Stärke, Dextrine, Guar, Johannisbrotkernmehl etc. beigegeben werden.

Im Wesentlichen werden diese homogenen, wässrigen, thermoreversibel gelierenden Biopolymer-Lösungen aus Flachdüsen bei Normaldruck gegossen. Durch die Reduktion des Wasseranteils in den Biopolymerlösungen und Erhöhung des Extrusionsdruckes gelingt es, auch viskosere Schmelzen zu giessen. Allerdings ist es auch dann vorgängig notwendig, eine homogene wässrige Lösung aus dem Biopolymer, Weichmacher und Zuschlagstoffen/Verdickungsmittel herzustellen. Dieser Prozess, welcher am einfachsten in einem Schmelztank (bis 6 bar, bis 130°C) wirtschaftlich realisierbar ist, ist wegen der hohen Viskosität nur ab mindestens ca. 30-40% Gesamt-Wasseranteil der verarbeitbaren Schmelze realisierbar.

Durch Verwendung integrierter Misch-Heiz- und Form-Anlagen, wie sie ein Ein- oder Zwei wellen-Extruder mit gekoppelter Flachdüse darstellt, können thermoplastische Massen bei erhöhten Drücken (50-300 bar) und erhöhten Temperaturen (80-300°C) zu Filmen verarbeitet werden. Die Anwesenheit von (temporärem oder permanentem) Weichmacher während der Verarbeitung zu einer homogenen Schmelze und Formung zu einem Film ist - abhängig von den Eigenschaften des Polymers selbst - unter Umständen unnötig, bzw. kann auf die Menge beschränkt werden, die für die mechanischen Eigenschaften des Materials in kaltem Zustand notwendig sind.

So kann Stärke wasserfrei oder mittels hohen Anteilen an Weichmachern (EP-1 103 254) durch Extrusion zu einem Produkt mit den für die Kapselformung sowie die Endapplikation erforderlichen mechanischen Eigenschaften (E-Modul, Bruchdehnung) verarbeitet werden, ohne dass dabei Gelbildner in wässriger Lösung wie Gelatine, Carrageenane oder andere Biopolymere vorhanden sein müssen.

Es hat sich aber in der Praxis gezeigt, dass derartige Massen ein unbefriedigendes Schlagzähigkeitsverhalten zeigen. Die Schlagzähigkeit einer plastifizierten Stärke wie in der EP-A-1 103 254 beschrieben ist für den praktischen Gebrauch als Hüllmaterial von Kapseln nur beschränkt ausreichend. Dies gilt sowohl für hochamylose- wie auch hochamylopektinhaltige Stärken. Zwar lassen sich durch die Auswahl des Weichmachers und die Einstellung der Gleichgewichtsfeuchte auf die mittlere kinetische Feuchte in Innenräumen der Marktklimazone (z.B. Klimazone 1: 19-20°C, 45%RH) und der sich daraus ergebenden Absenkung der Glasstemperatur Tg die mechanischen Eigenschaften verbessern. Auch sind die entsprechenden Formkörper, bedingt durch die geringen Abmessungen und Gewichte (welche ca. auf 100-2000 mg Gesamtgewicht und einem Gesamtvolumen von 0,2 - 2 ml beschränkt sind), nur begrenzt mechanischem Druck ausgesetzt. Bei Absenkung der Temperatur und/oder bei Entzug der Feuchte ist indes die Schlagzähigkeit kaum mehr ausreichend für den Verwendungszweck als Weichkapsel mit flüssigem Inhalt.

Dies liegt daran, dass Formkörper für die Anwendung als Nahrungsergänzungsmittel oder Arzneimittel, besonders auch für die orale Einnahme, in der Verpackung auf dem Transport- und Verteilungsweg folgenden typischen mittleren physikalischen Belastungen ausgesetzt sind: Während der Lagerhaltung in Innenräumen wirken in der Regel Temperaturen von 15-30°C bei einer Luftfeuchtigkeit von etwa 20-75%RH auf die Formkörper in verpackter Form ein. Beim Transport in einem nicht klimatisierten Fahrzeug wirken in der Regel Temperaturen von 0-35°C bei einer Luftfeuchtigkeit von etwa 50-90% auf die Formkörper ein. Diese Richtwerte können noch überschritten werden, z.B. bei unsachgerechter Lagerung in einem Kühlschrank oder in einem überhitzten Fahrzeug.

Zudem müssen derartige Formkörper einer mechanischen Kraft von ca. 30 N (wie sie beim Herausdrücken des Formkörpers aus einer Blisterverpackung auftreten) oder einer Schockbelastung von 0,05 Nm/s (beim Herunterfallen einer Kapsel aus etwa 1,5 m) standhalten können.

Bisherige Versuche zur Erhöhung der Schlagzähigkeit führten nicht zum gewünschten Erfolg. So ist in der EP-A-1 258 242 die Herstellung von thermoplastischen Massen mittels kontrollierter Steuerung der Feuchtigkeit der Masse beschrieben. Wenngleich gewisse mechanische Eigenschaften dadurch verbessert werden konnten, hat es sich doch gezeigt, dass trotz ausreichend tiefem Glasübergang von mindestens 30°C unter der Gebrauchstemperatur die Schlagzähigkeit der Stärke nur unwesentlich verbessert werden konnte.

Auch die Verwendung von hochamylosehaltiger oder hoch-amylopektinhaltiger Stärke konnte die Schlagzähigkeit der thermoplastischen Stärke nicht befriedigend optimieren.

Mischungen aus Stärke und anderen Biopolymeren wie Carrageenanen sind im Stand der Technik bereits zur Herstellung von Formkörpern vorgeschlagen worden. Bereits in der EP-A-1 103 254 sind als mögliche Zuschlagstoffe zur Grundmasse der thermoplastischen Stärke allgemein die "Gruppe der physikalisch und/oder chemisch modifizierten Biopolymere umfassend Cellulose, insbesondere teilhydroxypropylierte Cellulose, Alginate, Carageenan, Galactomannane, Glucomannane, Casein aufgelistet. Es fehlen aber entsprechende Ausführungsbeispiele, welche einen eventuellen Vorteil derartiger Mischungen zeigen würden.

In der US 6,340,473 und der US 6,949,256 wird Stärke als polymerer Füller mittlerer Molekülmasse zusammen mit dem Gelbildner Carrageenan und einem Weichmacher in einer wässrigen Lösung zur Herstellung von Formkörpern eingesetzt. In der US-6,949,256 wird hierzu eine Mischung aus iota- und kappa-Carrageenan vorgeschlagen, um die bei der alleinigen Verwendung von kappa-Carrageenan auftretenden Probleme zu überwinden. Es werden hohe Wassermengen von deutlich über 20 Gew.-% eingesetzt. In der US-6,340,473 werden Hüllmaterialien für Weichkapseln beschrieben, welche neben modifizierter Stärke einen hohen Anteil von 12-24 Gew.-% jota-Carrageenan enthalten.

Auch in der EP-A-1 448 608 werden Mischungen mit einem hohen Anteil von mehr als 60% kappa-Carrageenan beschrieben, wobei der Wasseranteil an der Gesamtmischung 50-95%, bevorzugt aber 60-85% beträgt.

In der US-4,859,484 beschreibt die Verarbeitung von Stärke mit Hydrokolloiden wie Guar Gummi und Johannisbrotkernmehl nach Vorquellung in Wasser, so dass Verarbeitungs-Wasseranteile von >200% resultieren. Auch hier werden vergleichsweise hohe Anteile an Gummen gelehrt.

Es ist in den beiden letzten Dokumenten nicht beschrieben, dass homogene Gemische hergestellt wurden. Es ist dem Fachmann hinreichend bekannt, dass das Herstellen von wässrigen Lösungen von hochpolymeren Stoffen wie Carrageenan, Gellan, Celluloseäthern und andern wasserlöslichen Biopolymeren sehr kritisch von der Wassertemperatur, der Wasseraktivität und der Korngrösse des Polymers abhängt. So wird meist empfohlen, eine Benetzung des Biopolymers mit nicht wässrigen Flüssigkeiten (wie z.B. Glycerin) oder Wasser von niedriger Temperatur vorzunehmen, oder das Biopolymer extrem schnell in Wasser zu dispergieren. Biopolymere in konzentriertem trockenem Zustand bilden extrem starke Wasserstoffbrücken. Bei einem Wasserkontakt ist somit zunächst kein Löse-, sondern nur ein Quellverhalten festzustellen. Je nach Wasserangebot bilden sich an den Polymerketten einfache Hydratisierungen (Viele Wasserstoffbrücken des Biopolymers bleiben noch bestehen). Erst bei sehr viel höherem Hydratisierungsgrad ("Mehrfachschichten von Wasser zwischen den Ketten") nimmt die Beweglichkeit des Polymers zu. Bei thermoreversiblen Gelen bleiben gewissen Restbindungen (bei Carrageenan helikale Strecken) in kaltem Zustand sogar bei extrem hohen Wasserverdünnungen erhalten. Es ist daher für den Fachmann nicht unbedingt klar, wie ein Gemisch aus Stärke, Weichmacher und Biopolymer zu einer homogenen thermoplastischen Masse aufbereitet werden kann.

Die Verarbeitung von Lösungen von Carrageenan oder anderen Hydrokolloiden zu komplexen Mischungen mit Stärke, mikrokristalliner Cellulose, oder Lactose im Granulationsprozess mit Hilfe von "Extrudern" (wie zum Beispiel "LCI twin dome" Granulatoren zu Pellets) sind für diese Erfindung nicht massgebend, denn bei den in der pharmazeutischen Industrie unter dem Namen "Extrusion" bekannten Vorgängen handelt es sich zwar oft auch um Apparaturen mit zwei Schnecken, die pulverige und kristalline Gemenge sehr effizient mischen bzw. mit flüssigen und viskosen Bindemittel-Lösungen vermischen können. Diese Verarbeitungsschritte sind aber im Sinne dieser Erfindung nicht als "Schmelzextrusion" zu bezeichnen, da die Mehrheit der Stoffe nicht in den plastischen Zustand durch erhöhten Druck, erhöhte Temperatur und Scherkräfte versetzt werden, und keinesfalls aus diesen Verfahren ein "homogenes" Produkt resultiert. Die hier angewendeten Begriffe für erhöhte Temperatur, erhöhten Druck und kurze Verweilzeit treffen für solche Granulierverfahren nicht zu.

Die EP-A-1 105 107, EP-A-1 105 108 und WO 2004/091533 beschreiben die Herstellung von Formkörpern, nämlich permanenten Weichmacher enthaltende Kapseln, welche mit dem Rotary Die-Verfahren hergestellt wurden. Dabei ist essentiell, dass sich die zunächst hergestellte heisse wässrige Lösung durch Sol→Gel-Übergang verfestigt und so der Verkapselungsmaschine zugeführt werden kann. Weiterhin ist wichtig, dass die heisse wässrige Lösung (Sol) möglichst viel an einer die Verarbeitung und mechanischen Eigenschaften im späteren Trockenzustand nicht störenden Zuschlagmasse enthält, da dies die mechanische Stabilität im Gelzustand erhöht und ein Versiegeln der beiden Bänder erst ermöglicht. Der scharfe Sol→Gel-Übergang einer reinen wässrigen Carrageenan-Lösung wird durch solche Zusätze breiter bzw. abgedämpft, so dass leichte Temperaturunterschiede zwischen Bandinnenseite und Bandaussenseite eine saubere Nahtbildung unter kleinem Inkrementen an Temperatur und Druck ermöglichen. Solche Systeme enthalten kappa-Carrageenane, iota-Carrageenane und/oder kappa-II-Carrageenane.

Keines der vorstehend genannten Dokumente beschreibt vorteilhafte Schlagzähigkeitseigenschaften der darin offenbarten Zusammensetzungen. Keines der Dokumente betrifft durch Schmelzextrusion hergestellte Massen aus Stärke und einem Biopolymer wie einem Carrageenan.

Es war die Aufgabe der vorliegenden Erfindung, thermoplastische stärkehaltige Massen als filmbildendes Material für die Herstellung von Formkörpern wie Weichkapseln bereitzustellen, welche bei unterschiedlichen Umgebungsbedingungen eine zufriedenstellende Schlagzähigkeit aufweisen und auf einfache Weise herstellbar sind.

Diese Aufgabe wird gemäss der vorliegenden Erfindung gelöst durch eine homogene, ungetrocknete, schmelzextrudierte thermoplastische Masse, enthaltend 30-60 Gew.-% Trockensubstanz an nativer oder chemisch modifizierter Stärke, höchstens 11 Gew.-% Trockensubstanz mindestens eines weiteren Biopolymers ausgewählt aus der Gruppe bestehend aus Carrageenan oder einem anderen Polysaccarid oder einem Protein, mindestens einen Weichmacher und maximal 20 Gew.-% zugesetztem Wasser.

Es hat sich überraschend gezeigt, dass derartige Massen eine vergleichsweise hohe Schlagzähigkeit aufweisen und diese auch bei Temperaturerhöhung und Feuchtigkeitsentzug (geringere Luftfeuchtgkeit in der Umgebung) in befriedigendem Masse beibehalten.

Die Verarbeitung von Stärke-Carrageenan-Massen unter wasserarmen Bedingungen ist bisher nicht beschrieben worden. Ebenso ist in dem oben genannten Stand der Technik jeweils ein sehr spezifisches Carrageenan mit/ohne stabilisierendes Puffersystem genannt, welche erfindungsgemäss nicht vorhanden sein muss.

Völlig überraschend wurde gefunden, dass mit der wasserarmen thermoplastischen Verarbeitung bei erhöhtem Druck und erhöhter Temperatur mit kurzer Verweildauer von Stärke zusammen mit Biopolymeren, insbesondere von Carrageenanen, schlagzähe Massen erhalten werden können.

Nach oben genanntem Stand der Technik werden Stärke-Carrageenan-Mischungen in Form von thermoreversibel gelierenden Massen mit hohem Wasseranteil bei Temperaturen von 60-80°C verarbeitet. Derartige Massen weisen bei Verarbeitung eine dynamische Viskosität um 10⁴ Pa·s auf. Die erfindungsgemäss beschriebene Masse dagegen besitzt bei Verarbeitungsbedingungen eine dynamische Viskosität im Bereich 10⁹ Pa·s und ist nach obiger Methode (Giessen von Folien) nicht verarbeitbar. Obwohl die Zusammensetzung der bekannten Stärke-Carrageenan-Mischungen (nach Trocknung) der erfindungsgemäss beschriebenen Masse ähnlich sein kann, handelt es sich um nicht identisches Material. Es kann auf die unterschiedliche Prozessführung zurückgeführt werden, dass das erfindungsgemäss beschriebene Material eine andere Mikrostruktur und entsprechend unterschiedliche mechanische und optische Eigenschaften aufweist.

Unter dem Begriff "homogen" bzw. "homogenisiert" soll im Sinn der vorliegenden Erfindung ein durch Schnmelzextrusion hergestelltes Material oder eine Masse verstanden werden, welche(s) an jeder Stelle im Material die im wesentlichen gleiche chemische und physikalische Zusammensetzung und Beschaffenheit aufweist. Zu geringfügigen Abweichungen kann es an den jeweiligen Material- oder Formteiloberflächen durch Aufnahme von Luftfeuchtigkeit kommen. Die im Extruder hergestellten erfindungsgemässen Massen zeichnen sich zudem in der Regel durch eine leichte Trübung aus. Diese Trübung ist ein Zeichen für "mikroskopische Inhomogenitäten" auf molekularer Ebene. Dies erklärt sich dadurch, dass durch das Aufschmelzen von nativen Stärkekörnern bzw. Granulatkörnern von prägelierter Stärke unter erhöhtem Druck und Temperatur und bei definierter Wasser-/Weichmacheraktivität zwar auf makroskopischer Ebene eine homogene Verteilung der Stärke- und Weichmachermoleküle erfolgt, auf mikroskopischer beziehungsweise molekularer Ebene hingegen sogenannte Knotenpunkte aus 1,4-Poly-Glucose-Doppelhelixen beziehungsweise sogenannte Blocklets übrig bleiben dürften (siehe Donald, Perry, Waigh: "The impact of internal granule structure on processing and properties", in Barsby, Donald, Frazier (eds.), Starch: Advances in structure and function, Royal Society of chemistry, Cambridge 2001). Eine (makroskopisch) homogene Mischung aus thermoplastisch aufgeschmolzenen "Körnern" von Stärke und Biopolymer kann deshalb auf molekularer Ebene aus Domänen mit mehrheitlich einer Komponente, sowie Domänen mit homogen vermischten Anteilen beider Komponenten, sowie durchlaufenden Molekülketten von Knotenpunkt zu Knotenpunkt bestehen.

Ohne an eine Erklärung gebunden sein zu wollen, vermuten die vorliegenden Erfinder, dass diese Domänen (mikroskopischen Inhomogenitäten) als sogenannte "Knautschzonen" dienen könnten, durch welche eine Schlagzähigkeitserhöhung bewirkt wird.

Im Sinne dieser Erfindung bezeichnet der Begriff "Schmelzextrusion" ein Verfahren, bei dem die Mehrheit der eingesetzten Stoffe aufgeschmolzen und durch erhöhten Druck, erhöhte Temperatur und Scherkräfte in den plastischen Zustand versetzt werden.

Unter wasserarm im Sinne dieser Erfindung ist zu verstehen, dass das Biopolymer und die Stärke mit Wasseranteilen (Feuchten) entsprechend einer Lager- und Vermarktungs-Gleichgewichtsfeuchte dosiert werden (dies heisst ca. 6-12% für Carrageenane, 8-15% für vorverkleisterte Stärke und 13-22% für native Stärken entsprechend einer Gleichgewichtsfeuchte von aw = 0.30 bis 0.60 (30-60% RH)), und für die eigentliche Verarbeitung im Zweiwellen-Extruder bei erhöhter Temperatur und erhöhtem Druck und nur einer geringen Verweildauer Wasseranteile von weniger als 20% zugesetzt werden. Der Gesamtwassergehalt aus den in den Komponenten als Gleichgewichtsfeuchte enthaltenem Wasser und zugesetztem Wasser beträgt weniger als 45%, noch bevorzugter weniger als 35%.

Als erhöhte Temperatur im Sinne dieser Erfindung gelten Temperaturen von mindestens 40°C über Raumtemperatur, also Produkttemperaturen von 60-150°C. Die Temperatur der Extruder-Heizsegmente sind als in etwa der Produkttemperatur entsprechend anzunehmen.

Als erhöhter Druck im Sinne dieser Erfindung sind Produkt-Drücke von 10 bis 300 atm zu verstehen. Der Druck an der Extruder-Ausgangsöffnung (Düse) ist dabei als etwa dem maximalen Produktdruck entsprechend anzunehmen.

Als erhöhte Scherleistung im Sinne dieser Erfindung ist eine Verarbeitung der erfindungsgemässen Massen, vorzugsweise in einem Zweiwellenextruder, bei einem Energieeintrag von 2 bis 10 kWh zu verstehen. Dies entspricht einer spezifischen Energie von etwa 0.15 bis 0.7 kWh/kg zu verarbeitender Masse. Die spezifische Scherenergie (shear stress) beträgt gemäss der vorliegenden Erfindung typischerweise zwischen 100.000 und 500.000 Pa.

Die vorliegende Erfindung betrifft eine ungetrocknete Masse. Darunter ist eine Masse zu verstehen, welche bei ihrer Herstellung und/oder Verarbeitung keine Trocknungsstufe durchläuft. Gemäss der vorliegenden Erfindung wird dann von einer Trocknungsstufe gesprochen, wenn sich der aw-Wert des homogenen Materials während dieser Verfahrensstufe um mehr als 0,1 verringert. Der aw-Wert (die Wasseraktivität eines Systems) ist definiert als die Flüchtigkeit von Wasser aus diesem System geteilt durch die Flüchtigkeit von reinem Wasser.

Als geringe Verweildauer im Sinne dieser Erfindung wird eine Verweilzeit zwischen erstem Kontakt aller Komponenten nach Dosierung in den Extruder und dem Austritt der geschmolzenen homogenen Mischung aus dem Extruder von maximal 5 Minuten, bevorzugt 3 Minuten, noch bevorzugter 2 Minuten verstanden.

Unter Schlagzähigkeit ist im Rahmen der vorliegenden Erfindung die Schlagzugzähigkeit nach DIN EN ISO 8256 zu verstehen. Dabei werden Filme (Bänder) von ca. 400-800 µm Dicke, wie sie für die Herstellung von Weichkapseln nach dem Rotary Die-Verfahren verlangt werden, konditioniert, zu Prüflingen ausgestanzt und durch ein Schlagpendel bei festgelegten Schlaggeschwindigkeiten auf Sprödigkeit oder Zähigkeit bei hoher Zugverformungsgeschwindigkeit untersucht. Hierbei ist das Versagensverhalten an Weichkapseln bei verschiedenen Klimabedingungen (wie warm/trocken, kalt/feucht, normal oder warm/feucht) zu prüfen.

Gemäss der vorliegenden Erfindung werden Massen aus Stärke und mindestens einem weiteren Biopolymer eingesetzt.

Unter Stärke im Sinn dieser Erfindung sind lineare 1,4-Polyglucane (Amylose), verzweigte 1,6-//1,4 Polyglucane (Amylopektin) bzw. deren Kombination zu einem sehr grossen verzweigten Polymer mit einem gewicht von bis zu mehr als 1 Mio. Dalton zu verstehen, wie sie als so genannte native Stärken aus Mais, Waxy Mais, Reis, Kartoffel, Tapioka (Manihot), Arrowroot, Sorghum, Hirse, Hafer, Weizen, Hartweizen, Roggen, Gerste, Buchweizen, Erbse, Linse, Bohne, Mondbohne, Mungobohne, Erdnuss, Maranta, Kurkuma, Canna, Perlsago, Kastanie, Banane, Dioscorea, Batate oder andern Pflanzen gewonnen werden können.

In einer bevorzugten Ausführungsform wird die Stärke aus den nativen Stärken von Kartoffel, Mais, Waxy Mais, Reis und Tapioka ausgewählt.

Weiter werden erfindungsgemäss unter Stärke auch die so genannten vorverkleisterten oder kaltwasserlöslichen Stärken verstanden, deren natürliche Kornstruktur durch Verquellen in Wasser und Erhitzen mehrheitlich zerstört, zu einer strukturlosen, stark wasserhaltigen Masse, und anschliessend durch Trocknen über Walzen, Versprühung oder ähnlichen Verfahren zu einem pulverigen, körnigen oder flockigen Zustand verarbeitet wurden. In einer bevorzugten Ausführungsform ist die Stärke eine vorverkleisterte Kartoffel oder Maisstärke.

Weiter werden erfindungsgemäss unter Stärke auch die chemisch modifizierten Stärken verstanden, deren Polysaccharidketten durch Einführung einer oder mehrere Modifikationen wie Hydroxypropylierung, Acetalisierung, Phosphatidierung oder durch Oxidation verändert wurden. In einer bevorzugten Ausführungsform ist die Stärke eine hydroxypropylierte Kartoffel- oder Tapiokastärke.

Zusätzlich zu Stärke enthalten die erfindungsgemässen Massen mindestens ein weiteres Biopolymer, ausgewählt aus der Gruppe bestehend aus Carrageenan, Gellan oder einem Protein.

Unter Carrageenan im Sinne dieser Erfindung werden langkettige, lineare, anionische Hydrokolloide (Polysaccharide, aus verschiedenen Rotalgenarten verstanden, insbesondere aus Knorpeltang (Chondrus crispus), Eucheuma spinosum (iota-Carrageenan), Kappaphycus cottonii (kappa-Carrageenan) nach entsprechende Extraktion im alkalischen Milieu und Fällung mit Ethanol.

Gemäss der vorliegenden Erfindung sind aber auch die PES (*Processed Eucheuma Seaweed*) umfasst. Hierbei handelt es sich um semi-refined Carrageenane (E407a), die direkt durch alkalische Extraktion (ohne Ethanol-Fällung) hergestellt werden.

Die verschiedenen Carrageenan-Typen unterscheiden sich in erster Linie durch den Anteil an Galaktose und 3,6-Anhydrogalaktose sowie über die Anzahl an vorhandenen Sulfatgruppen. Bekannt sind als gelbildenden Carrageenane nur └κ- und 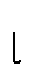 -Carrageenan, während das λ-Carragenan nur verdickend wirkt und die µ- und ν-Carrageenane als Vorstufen zu κ- und 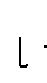 -Carrageenan betrachtet werden können, da sie bei der alkalischen Extraktion weitestgehend in diese Typen umgewandelt werden. Es sind auch kappa-iota-Hybrid-Carrageenane beschrieben worden. Zudem wurde kappa-II-Carrageenan in gegossenen Weichkapselfilmen verwendet. Der Anteil der Carrageenan-Typen im fertigen Carrageenan ist also sowohl von der verwendeten Algenart als auch von dem Herstellungsprozess abhängig. Aus diesem Grund handelt es sich bei kommerziellem Carrageenan auch nie um absolut reine einzelne Typen.

κ-Carrageenan geliert mit Kalium-Ionen in wässriger Lösung zu einem festen und spröden Gel, während es sich mit Calcium-Ionen zu einem festen, elastischen und synäresearmen Gel umwandelt. iota-Carrageenan geliert mit Calciumionen in wässriger Lösung. Auch iota-Carrageenan ist nur als Natriumsalz kaltlöslich und benötigt als Calcium- oder Kaliumform ebenfalls höhere Temperaturen.

Anstelle von Carrageenanen können gemäss der vorliegenden Erfindung aber auch andere Biopolymere eingesetzt werden. Es handelt sich hierbei beispielhaft um
- Polysaccharide vom Typ
   a) Gelbildner, wie Agar (*Gracilaria-, Gelidiopsis-*, *Gelidium-, Hypnea-* und *Sphaerococcus*-Arten), Gellan, Hsian-tsao (aus mesone procumbens), Curdlan (beta(1,3)-glucan), und Furcellan (aus Furcellaria fastigiata),
   b) spezifische Stärkeabbauprodukte und Modifikationen, wie Pullulan (Polymaltotriose),
   c) Polysaccaride aus Früchten wie Johannisbrotkernmehl (Carob, GM= Galactomannan), Guar (GM), Tarakernmehl, psyllium seed gum, und Konjac (Glucomannan),
   d) Baumsaft-Gummis (Exsudate) wie: Arabisches Gummi, Tamarindengummi, Khaya grandifolium Gummi, Ghatti (aus Anogeissus Lati-folia), Tragacanth (aus Astralgus Arten), und Karaya (aus Sterculia Arten),
   e) Pectine (methylierte poly-galacturone),
   f) Alginate (poly-Mannuron /Gulurone) und deren Salze (die auch mit divalenten Kationen gelieren können),
   g) Exozelluläre Polysaccharide von Mikroorganismen, wie Xanthan (Beta-1,4-glucan) (B-D-Glucose, a-D-mannose and a-D glucoronsäure 2:2:1), Scleroglucan (Aus *Sclerotium rolfsii*), Schizophyllan (aus *Schizophyllan commune*), Succinoglycan (von *Rhizobium meliloti*), Rhamsan (von *Sphingomonas paucimobilis)*, Welan, und Sphingan,
- Polyaminosaccharide wie Chitosan (Beta-1,4, Poly-D-glucosamin) und Hyaluronan (Glycosaminoglycan)
- Proteine wie
   a) pflanzliche Proteine (auch fraktioniert) aus Soja, Weizen, Hafer, Gerste, Roggen, Kartoffel, Erbse, und Mais beziehungsweise deren Mehle (d.h. Stärke plus Protein in natürlichem Gemisch),
   b) tierische Proteine wie Kasein, Milchprotein, Eialbumin

Erfindungsgemäss können auch Gemische verschiedener Biopolymere verwendet werden, wobei die Gesamtmenge an Biopolymer in den angegebenen Bereichen zu liegen hat.

Gemäss der vorliegenden Erfindung sind die Stärke und das weitere Biopolymer in einem bestimmten Verhältnis zueinander einzusetzen. Die Stärke ist in einer Menge von 30-60 Gew.-% Trockensubstanz, vorzugsweise 33-55 Gew.-% Trockensubstanz einzusetzen. Das zusätzliche Biopolymer ist in einer Menge von höchstens 11 Gew.-% Trockensubstanz, vorzugsweise 2 bis höchstens 11 Gew.-% Trockensubstanz, noch bevorzugter 3 bis 10,5 Gew.-% Trockensubstanz einzusetzen.

Unter Trockensubstanz ist erfindungsgemäss die Menge der entsprechenden Substanz im Trockenzustand (d.h. ohne die üblicherweise vorhandene, vorstehend beschrieben Gleichgewichtsfeuchte) zu verstehen.

Die erfindungsgemässen Massen enthalten zudem mindestens einen permanenten Weichmacher. Unter permanentem Weichmacher im Sinne dieser Erfindung werden kurzkettige Stoffe(d.h. Stoffe mit einem Molekulargewicht von <1000 Dalton) verstanden, die einen hohen Löslichkeits-Parameter besitzen und eine Schmelzpunktserniedrigung der Stärke bewirken. Das Löslichkeits-Parameter-Konzept wurde von Hildebrand und Scatchard vorgeschlagen und vor allem im Bereich der Polymere weiterentwickelt (Siehe z.B. Handbook of sulubility parameters and other cohersion parameters, 2nd edition, A.F.M. Barton ed, CRC Press, Boca Raton,(1991)). Solche Weichmacher sind bereits aus der US-5,362,777 bekannt. Besonders bevorzugt sind Weichmacher, welche als Lebensmittelzusatzstoffe zugelassen sind oder zumindest als pharmazeutische Hilfsstoffe keine negativen gesundheitlichen Wirkungen aufweisen. Solche Weichmacher werden ausgewählt aus der Gruppe bestehend aus 1,2-Propylenglykol, 1,3-Propylenglykol, Glycerin, niedrigen Polyethylenglykolen (PEG), Polyglycerinen, Sorbitol, Maltitol, Erythritol, Xylitol, Mannitol, Isomaltitol, Lactitol, Maltotriitol, hydrierten Oligosacchariden, Sorbitanen, Glucose, Glucosesirup, Dianhydrosorbit, Isosorbiden, Maltol, Isomaltol, Maltodextrin, N-Methylpyrrolidon, Triethylcitrat und Glycerintriacetat.

Bevorzugt im Sinne dieser Erfindung sind Weichmacher, die auf Grund ihres Molekulargewichts und Dampfdruckes möglichst wenig Migration zeigen, mit den Hydroxylgruppen der Stärke und Biopolymere eine hohe Wechselwirkung eingehen, sowie eine geringe Kristallisationsneigung haben, physiologisch unbedenklich sind, und eine geringe Sorption bei Umgebungsfeuchten von mehr als 30% RH und eine hohe Sorption bei Feuchten von weniger als 30% RH haben. Bevorzugt sind diese Weichmacher ausgewählt aus der Gruppe bestehend aus Glycerin, Sorbitol, Maltitol und hydrierten Stärkeabbauprodukten.

Erfindungsgemäss ist der mindestens eine Weichmacher in der Masse in einer Menge von 20 bis 45 Gew.-% Trockensubstanz, vorzugsweise 25 bis 45 Gew.-% Trockensubstanz vorhanden.

Der erfindungsgemässen Masse können zudem noch weitere gängige Zusatzstoffe zugesetzt sein. Derartige Zusatzstoffe sind dem Fachmann bekannt. Beispielsweise kann ein internes Gleit- und Formtrennmittel zugegeben werden, welches ausgewählt ist aus der Gruppe bestehend aus Lecithinen, Mono-, Di- oder Triglyceriden von Speisefettsäuren, Polyglycerinester der Speisefettsäuren, Polyethylenglycolester der Speisefettsäuren, Zuckerester der Speisefettsäuren und Speisefettsäuren.

Unter Speisefettsäuren werden die als Säurekomponenten der Triglyceride natürlicher Fette vorkommenden Monocarbonsäuren verstanden. Sie weisen eine gerade Anzahl von C-Atomen auf und haben ein unverzweigtes Kohlenstoffgerüst. Die Kettenlänge der Fettsäuren variiert von 2 bis 26 C-Atomen. Eine grosse Gruppe der Fettsäuren sind gesättigte Fettsäuren.

Das Gleit- und Formtrennmittel ist in der Mischung bevorzugt in einem Bereich von 0 bis 4 Gew.% bezogen auf das Gesamtgewicht der Mischung enthalten. In einer bevorzugten Ausführungsform enthält die Mischung Glycerinmonostearat.

Weiterhin kann der Mischung noch mindestens ein Zuschlagstoff in einem Gewichtsbereich von 0,1 Gew.% bis 15 Gew.%, bevorzugt von 0,1 Gew.% bis 5 Gew.% bezogen auf das Gesamtgewicht der Mischung zugesetzt werden. Die Zuschlagstoffe sind ausgewählt aus der Gruppe bestehend aus Carbonaten und Hydrogencarbonaten der Alkali- und Erdalkaliionen, weiteren Zerfallshilfen, Füllstoffen, Farbstoffen, Antioxidantien, oder physikalisch und/oder chemisch modifizierte Biopolymere, insbesondere Polysacchariden und pflanzliche Polypeptiden.

Die Opazität der homogenisierten Masse wird z.B. bevorzugt mit dem Zusatz von Titaniumdioxid oder Eisenoxiden (oder ähnlichen Stoffen) als Füllstoff erreicht.

Als Zerfallshilfe für einen schnellen Zerfall der Kapselhülle werden bevorzugt Calciumcarbonat und Amylasen zugesetzt.

Wie vorstehend ausgeführt zeichnen sich die erfindungsgemässen Massen dadurch aus, dass nur maximal 20 Gew.-% Wasser von aussen zugesetzt wird (d.h. zusätzlich zum Feuchtigkeitsgehalt der anderen Komponenten). Eine derart geringe Wassermenge kann nur dadurch realisiert werden, dass die erfindungsgemässen Massen durch ein besonderes Schmelzextrusionsverfahren hergestellt werden.

Die Verarbeitung der einzelnen Komponenten zu homogenen, thermoplastischen Massen erfolgt erfindungsgemäss in einer für die gleichzeitige Mischung, Erwärmung und Scherung aller Komponenten brauchbaren Einrichtung, wie sie ein Zwei- oder Einwellenextruder darstellt. Derartige Geräte sind dem Fachmann hinreichend bekannt.

Auch unterscheidet der Fachmann den Gebrauch eines Extruders für innige Vermischung, Scherung und Aufschmelzen klar von der Nutzung eines Extruders lediglich als Pumpe für eine hochviskose Masse zur Förderung und Erzielung eines gleichmässigen Druckes. Der Fachmann wird die Konfiguration einer Schnecke in einem Einwellenextruder deshalb zum Aufschmelzen und Transportieren völlig anders gestalten wie das gegen- oder gleichlaufende Schneckenpaar in einem Zweiwellenextruder zur Herstellung von Massen.

Auf die gleiche Art legt ein Fachmann die Prozessbedingungen fest, die zur Erreichung einer luftfreien, homogenen thermoplastischen Masse notwendig sind. Die Fördermenge, Durchlaufzeit, das Temperaturprofil und die spezifische Scherung sind exakt einzustellen, um brauchbare Resultate zu erzielen.

Um die für den Schmelz- und Knetvorgang notwendige Energie (Temperatur) und Scherleistungen einbringen zu können, ist erfindungsgemäss neben einer dafür geeigneten Schneckenkonfiguration auch ausreichende Verweilzeit sicherzustellen. Dazu sind besonders gleichlaufende (co-rotating) Zweiwellenextruder mit einer Länge (gemessen in Multiplen des Durchmessers) von mindestens L/D>20, bevorzugt >36, noch bevorzugter >40, geeignet.

Bei der Verarbeitung von Biopolymeren zu homogenen Massen war wie vorstehend ausgeführt bekannt, dass die hohe Scherung bei der Verarbeitung in Extrudern nur bei sehr hohem Wassergehalt ohne Schädigung des Polymers vorgenommen werden kann und die Wasseraktivität sehr hoch sein muss, um eine homogene und vollständige Verquellung zu ereichen. Es war daher selbst für den Fachmann völlig überraschend, dass unter bestimmten Verfahrensbedingungen die Verarbeitung auch unter sehr wasserarmen Bedingungen vorgenommen werden kann, um so direkt zu den erfindungsgemäss benötigten wasserarmen Produkten zu gelangen.

Gemäss der vorliegenden Erfindung wird das Verfahren in einem 2-Wellenextruder typischerweise bei
- Scherleistungen von 0.15 bis 0.70 kWh/kg Masse
- Einem Temperaturprofil von 100-130°C
- Einer Maximaltemperatur von 150°C
- Verweilzeiten von 1 bis 3 Minuten
- In Gegenwart eines permanenten Weichmachers und höchstens 20 Gew.-% zusätzlich zugegebenem Wasser
   durchgeführt. Dabei werden homogene thermoplastische Massen mit stark verbesserter Schlagzähigkeit gegenüber analogen Massen aus reinen Stärken erhalten.

Das Verfahren zur Herstellung der homogenen thermoplastischen Masse sowie die anschliessende Verarbeitung zu Weichkapseln im Rotary Die-Verfahren ist in seinen Grundzügen bereits in der EP-A-1 103 254 offenbart, auf deren entsprechenden Inhalt hiermit Bezug genommen wird. Gemäss der vorliegenden Erfindung sind aber die vorstehenden Verfahrensparameter einzuhalten.

Zudem werden gemäss der vorliegenden Erfindung die extrudierten Massenstränge zur schnellen Abkühlung in ein Kühlmedium eingeführt. Es handelt sich hierbei vorzugsweise um ein lebensmittelgerechtes, nicht flüchtiges, nicht umweltschädigendes, schwer entflammbares Kühlmedium, beispielsweise um mittelkettige Trigylceride (d.h. Trigylceride mit einer Kettenlänge im Fettsäureanteil von 6 bis 18 C-Atomen). Es wurde gemäss der vorliegenden Erfindung überraschend gefunden, dass durch Verwendung eines solchen Kühlmediums ein Abkühlen der erfindungsgemässen Masse möglich ist, ohne dass es hierbei zu einer Änderung des Feuchtigkeitsgehalts des Produkts kommt. Die Möglichkeit der Verwendung eines Kühlmediums unter Erhalt der Produktfeuchte für die Strangkühlung nach der 2-Wellenextrusion war für den Fachmann der Verarbeitung von Thermoplasten nicht offensichtlich, da die meisten bekannten Polymere wenig/keine Sorption zeigen und die gekühlte Luft in der Regel ausreicht. Wenn Thermoplasten in flüssige Kühlmedien eingetragen werden, wird dazu meist Wasser benützt, welches abgeblasen beziehungsweise abgetrocknet werden kann.

Ebenso ist für die Herstellung eines Bandes aus erfindungsgemässem Material die Verwendung einer Chillroll und zur Relaxation von Quer und Längsspannungen die Verwendung eines Relaxationsbades (ohne Veränderung des Wassergehaltes des Filmes) vorteilhaft. Auf die entsprechende Offenbarung der EP-A-1 249 219 wird ausdrücklich Bezug genommen.

Dei Verarbeitung erfindungsgemässer Massen direkt oder aus wiederaufgeschmolzenen Massen mittels Extrusion und Formung mit Flachdüsen zu einem Film und die Verwendung von 2 Filmen im Rotary-Die Verfahren zu Weichkapseln wurde bereits in EP-A-1 103 254 beschrieben. Auf die entsprechende Offenbarung wird ausdrücklich Bezug genommen.

Die vorliegende Erfindung wird nachstehend unter Bezug auf nicht einschränkende Beispiele näher erläutert.

### Beispiele

### Beispiel 1

Native Tapiokastärke (Novation 3600, National Starch), iota-Carrageenan (Satiagel USC150) oder Gellan (Kelcogel LT100), ein Weichmachergemisch aus Glycerin, Sorbitolsirup und Maltitolsirup, sowie Wasser wurden in den nachstehend angegebenen Mengen eingesetzt. Die Stärke und das entsprechende Biopolymer einerseits sowie die Weichmacher und das Wasser andererseits wurden separat vorgemischt und anschliessend mit je einer gravimetrischen Pulverdosiervorrichtung (das Gemisch aus Stärke und Biopolymer) und einer Flüssigdosiervorrichtung (Weichmache rund Wasser) in einen Zweiwellenextruder (Coperion ZSK 25, L/D=48) dosiert:

| **Komponente** | **Bsp. 1a Menge Feuchtsubstanz** | **Bsp. 1b Menge Feuchtsubstanz** | **Menge Trockensubstanz** |
|---|---|---|---|
| Tapiokastärke (11% Feuchtigkeit) | 41.24 | 41.24 | 36.70 |
| Iota-Carrageenan (Satiagel USC150, 6.6% Feuchtigkeit) | 5.02 | | 4.69 |
| Gellan (Kelcogel LT100, 10% Feuchtigkeit) | | 5.21 | 4.69 |
| Glycerin (0.5% Feuchtigkeit) | 10.68 | 10.68 | 10.63 |
| Sorbitolsirup (30% Feuchtigkeit) | 23.90 | 23.90 | 16.73 |
| Maltitolsirup (15% Feuchtigkeit) | 16.29 | 16.29 | 13.85 |
| Wasser | 2.87 | 2.68 | 17.40 |

Die derart hergestellten Mischungen wurden im Zweiwellenextruder gemäss folgenden Temperaturprofil in den verschiedenen Segmenten des Extruders aufgeschmolzen und zu einer homogenen, thermoplastische Masse verarbeitet:

### Temperaturprofil 2-Wellenextrusion

| T-1 | T-G2 | T-G3 | T-G4 | T-G5 | T-G6 | T-G7 | T-G8 | T-G9 | T-G10 | T-G11 | T-G12 | Düse |
|---|---|---|---|---|---|---|---|---|---|---|---|---|
| Einzug | (°C) | (°C) | (°C) | (°C) | (°C) | (°C) | (°C) | (°C) | (°C) | (°C) | (°C) | |
| RT | 100 | 100 | 130 | 150 | 150 | 130 | 120 | 110 | 100 | 100 | 100 | 95 |
| | | M | M | M | SS | SS | | | E | | | |

| | | | | | | | | | | | | |
|---|---|---|---|---|---|---|---|---|---|---|---|---|
| M=Mischen; SS= Starke Scherung, E= Entgasen | | | | | | | | | | | | |

Im Extruder wurden folgende Bedingungen angelegt:

| Beispiel | Durchsatz (kg/h) | Rotation Extruderschnecke (U/min) | Vakuum (mbar) | Drehmoment (%) | Düsendruck (bar) |
|---|---|---|---|---|---|
| 1a | 6.4 | 120 | 800 | 34 | 36 |
| 1b | 7.5 | 110 | 500 | 30 | 32 |

Es wurde ein vollständig homogenisiertes Material erhalten, welches beim Düsenaustritt soweit abgekühlt war, dass es nicht mehr schäumte.

Die Stränge wurden zur schnellen Abkühlung ohne Verlust von Feuchte in lebensmittelgerechtes Kühlmedium (mittelkettige Triglyceride) eingeführt, dann zu einem Granulat von ca. 2 x 3 mm geschnitten und in PE-Säcken feuchtedicht gelagert.

Die hergestellten Granulate wurden in einem Einwellen-Extruder (Collin, E 30 M (Schneckendurchmesser 30 mm, Schneckenlänge 25D, max. Drehmoment 350 Nm) gemäss dem nachstehenden Temperaturprofil extrudiert und in einer Flachdüse (FA. Verbruggen) zu einem Band von ca. 0.8 mm Dicke geformt

| Temperaturprofil Einwellenextrusion | | | | | | | | |
|---|---|---|---|---|---|---|---|---|
| T1 | T2 | T3 | T4 | Ad | D1 | D2 | D3 | Drehzahl |
| (°C) | (°C) | (°C) | (°C) | (°C) | (°C) | (°C) | (°C) | rpm |
| 115 | 135 | 135 | 135 | 125 | 120 | 120 | 120 | 65 |

Die erhaltenen Filme wurden in Klimakammern konditioniert und mittels einer Zugprüfmaschine (Instron 3345) bzw. einem Pendelschlagwerk (Zwick Typ B5102.202) vermessen.

Es wurden unter verschiedenen Konditionierungsbedingungen folgende Resultate erhalten:

| Konditionierungsbedingungen | Schlagzähigkeit (kJ/m²) | |
|---|---|---|
| | Bsp. 1a | Bsp. 1b |
| 25°C, 50% RH | >575 | 435 |
| 30°C, 20% RH | >530 | 125 |

### Beispiel 2

Native Tapiokastärke (Novation 3600, National Starch) oder native Kartoffelstärke (Emsize E9, Emsland), Sojaprotein oder Wiezenprotein, Glycerin oder ein Gemisch aus Glycerin und Sorbitolsirup, sowie Wasser und Glycerinmonostearat wurden in den nachstehend angegebenen Mengen eingesetzt. Die Stärke und das entsprechende Biopolymer einerseits sowie die Weichmacher, das Glycerinmonostearat und das Wasser andererseits wurden separat vorgemischt und anschliessend mit je einer gravimetrischen Pulverdosiervorrichtung (das Gemisch aus Stärke und Biopolymer) und einer Flüssigdosiervorrichtung (Weichmache rund Wasser) in einen Zweiwellenextruder (Coperion ZSK 25, L/D=48) dosiert:

| **Komponente** | **Bsp. 2a Menge Trocken-substanz** | **Bsp. 2b Menge Trocken-substanz** | **Bsp. 2c Menge Trocken-substanz** |
|---|---|---|---|
| Tapiokastärke (11% Feuchtigkeit) | 42.68 | | 52.20 |
| Kartoffelstärke (20.5% Feuchtigkeit) | | 50.12 | |
| Weizenprotein (4.4% Feuchtigkeit) | 10.16 | | |
| Sojaprotein (5.2% Feuchtigkeit) | | 6.88 | 6.67 |
| Glycerin (0.5% Feuchtigkeit) | 14.47 | 25.00 | 26.67 |
| Sorbitolsirup (30% Feuchtigkeit) | 13.93 | | |
| Glycerinmonostearat (0% Feuchtigkeit) | 0.32 | | |
| Wasser | 18.44 | 18.00 | 14.46 |

Die derart hergestellten Mischungen wurden im Zweiwellenextruder gemäss dem in Beispiel 1 angegebenen Temperaturprofil in den verschiedenen Segmenten des Extruders aufgeschmolzen und zu einer homogenen, thermoplastische Masse verarbeitet.

Im Extruder wurden folgende Bedingungen angelegt:

| Beispiel | Durchsatz (kg/h) | Rotation Extruderschnecke (U/min) | Vakuum (mbar) | Drehmoment (%) | Düsendruck (bar) |
|---|---|---|---|---|---|
| 2a | 9.4 | 140 | 800 | 47 | 58 |
| 2b | 10.6 | 120 | 700 | | 45 |
| 2c | 8.0 | 120 | 800 | 32 | 41 |

Es wurde ein vollständig homogenisiertes Material erhalten, welches beim Düsenaustritt soweit abgekühlt war, dass es nicht mehr schäumte.

Die Stränge wurden zur schnellen Abkühlung ohne Verlust von Feuchte in lebensmittelgerechtes Kühlmedium (mittelkettige Triglyceride) eingeführt, dann zu einem Granulat von ca. 2 x 3 mm geschnitten und in PE-Säcken feuchtedicht gelagert.

Die hergestellten Granulate wurden wie in Beispiel 1 beschrieben in einem Einwellen-Extruder extrudiert und in einer Flachdüse (FA. Verbruggen) zu einem Band von ca. 0.8 mm Dicke geformt

Die erhaltenen Filme wurden in Klimakammern konditioniert und mittels einer Zugprüfmaschine (Instron 3345) bzw. einem Pendelschlagwerk (Zwick Typ B5102.202) vermessen.

Es wurden unter verschiedenen Konditionierungsbedingungen folgende Resultate erhalten:

| Konditionierungs- | Schlagzähigkeit (kJ/m²) | | |
|---|---|---|---|
| bedingungen | Bsp. 2a | Bsp. 2b | Bsp. 2c |
| 25°C, 50% RH | 550 | 490 | 305 |
| 30°C, 20% RH | 500 | 310 | 362 |

### Beispiel 3

Native Tapiokastärke (Novation 3600, National Starch), kappa-Carrageenan, ein Gemisch aus Glycerin und Sorbitolsirup, Glycerinmonostearat sowie Wasser wurden in den nachstehend angegebenen Mengen eingesetzt. Die Stärke und das entsprechende Biopolymer einerseits sowie die Weichmacher, Glycerinmonostearat und das Wasser andererseits wurden separat vorgemischt und anschliessend mit je einer gravimetrischen Pulverdosiervorrichtung (das Gemisch aus Stärke und Biopolymer) und einer Flüssigdosiervorrichtung (Weichmache rund Wasser) in einen Zweiwellenextruder (Coperion ZSK 25, L/D=44) dosiert:

| **Komponente** | **Bsp. 3a Menge Trocken-substanz** | **Bsp. 3b Menge Trocken-substanz** |
|---|---|---|
| Tapiokastärke (11% Feuchtigkeit) | 46.74 | 42.68 |
| Kappa-Carrageenan (5.20% Feuchtigkeit) | 5.08 | 10.16 |
| Glycerin (0.5% Feuchtigkeit) | 14.47 | 14.47 |
| Sorbitolsirup (30% Feuchtigkeit) | 13.93 | 13.93 |
| Glycerinmonostearat (0% Feuchtigkeit) | 0.32 | 0.32 |
| Wasser | 19.46 | 18.45 |

Die derart hergestellten Mischungen wurden im Zweiwellenextruder gemäss dem in Beispiel 1 angegebenen Temperaturprofil in den verschiedenen Segmenten des Extruders aufgeschmolzen und zu einer homogenen, thermoplastische Masse verarbeitet.

Im Extruder wurden folgende Bedingungen angelegt:

| Beispiel | Durchsatz (kg/h) | Rotation Extruderschnecke (U/min) | Vakuum (mbar) | Drehmoment (%) | Düsendruck (bar) |
|---|---|---|---|---|---|
| 3a | 9.4 | 140 | 850 | 58 | 60 |
| 3b | 9.4 | 140 | 850 | 55 | 55 |

Es wurde ein vollständig homogenisiertes Material erhalten, welches beim Düsenaustritt soweit abgekühlt war, dass es nicht mehr schäumte.

Die Stränge wurden zur schnellen Abkühlung ohne Verlust von Feuchte in lebensmittelgerechtes Kühlmedium (mittelkettige Triglyceride) eingeführt, dann zu einem Granulat von ca. 2 x 3 mm geschnitten und in PE-Säcken feuchtedicht gelagert.

Die hergestellten Granulate wurden wie in Beispiel 1 beschrieben in einem Einwellen-Extruder extrudiert und in einer Flachdüse (FA. Verbruggen) zu einem Band von ca. 0.8 mm Dicke geformt

Die erhaltenen Filme wurden in Klimakammern konditioniert und mittels einer Zugprüfmaschine (Instron 3345) bzw. einem Pendelschlagwerk (Zwick Typ B5102.202) vermessen.

Es wurden unter verschiedenen Konditionierungsbedingungen folgende Resultate erhalten:

| Konditionierungs- | Schlagzähigkeit (kJ/m²) | |
|---|---|---|
| bedingungen | Bsp. 3a | Bsp. 3b |
| 25°C, 50% RH | - | - |
| 30°C, 20% RH | 141 | 179 |

### Vergleichsbeispiel 1

Eine stärkehaltige Masse ohne zusätzliches Biopolymer wurde in den nachstehend angegebenen Mengen eingesetzt und wie in den erfindungsgemässen Beispielen verarbeitet:

| **Komponente** | **Vgl-Bsp. 1a Menge Trocken-substanz** | **Vgl-Bsp. 1b Menge Trocken-substanz** |
|---|---|---|
| Tapiokastärke (11% Feuchtigkeit) | 48.07 | |
| Kartoffelstärke (18.0% Feuchtigkeit) | | 49.41 |
| Glycerin (0.5% Feuchtigkeit) | 7.76 | 4.96 |
| Sorbitolsirup (30% Feuchtigkeit) | 14.25 | 14.65 |
| Maltitolsirup (25% Feuchtigkeit) | 11.81 | 9.64 |
| Glycerinmonostearat (0% Feuchtigkeit) | 0.74 | 1.10 |
| Wasser | 17.37 | 20.26 |

Im Extruder wurden folgende Bedingungen angelegt:

| Beispiel | Durchsatz (kg/h) | Rotation Extruderschnecke (U/min) | Vakuum (mbar) | Drehmoment (%) | Düsendruck (bar) |
|---|---|---|---|---|---|
| Vgl.-Bsp. 1a | 14.0 | 110 | 500 | 55 | 60 |
| Vgl.-Bsp. 1b | 10.5 | 120 | 500 | 75 | 78 |

Es wurde ein vollständig homogenisiertes Material erhalten, welches beim Düsenaustritt soweit abgekühlt war, dass es nicht mehr schäumte.

Die Stränge wurden zur schnellen Abkühlung ohne Verlust von Feuchte in lebensmittelgerechtes Kühlmedium (mittelkettige Triglyceride) eingeführt, dann zu einem Granulat von ca. 2 x 3 mm geschnitten und in PE-Säcken feuchtedicht gelagert.

Die hergestellten Granulate wurden wie in Beispiel 1 beschrieben in einem Einwellen-Extruder extrudiert und in einer Flachdüse (FA. Verbruggen) zu einem Band von ca. 0.8 mm Dicke geformt

Die erhaltenen Filme wurden in Klimakammern konditioniert und mittels einer Zugprüfmaschine (Instron 3345) bzw. einem Pendelschlagwerk (Zwick Typ B5102.202) vermessen.

Es wurden unter verschiedenen Konditionierungsbedingungen folgende Resultate erhalten:

| Konditionierungs- | Schlagzähigkeit (kJ/m²) | |
|---|---|---|
| bedingungen | Vgl.-Bsp. 1a | Vgl.-Bsp. 1b |
| 25°C, 50% RH | 790 | 617 |
| 30°C, 20% RH | 60 | 50 |

### Vergleichsbeispiel 2

Eine Gelatine-Masse (Gelatine 165 bloom, Gelita) ohne zusätzliches Biopolymer wurde in den nachstehend angegebenen Mengen eingesetzt und wie in den erfindungsgemässen Beispielen verarbeitet:

| **Komponente** | **Vgl-Bsp. 2 Menge Trocken-substanz** |
|---|---|
| Gelatine (10% Feuchtigkeit) | 48.44 |
| Glycerin (0.5% Feuchtigkeit) | 17.68 |
| Wasser | 33.89 |

Dazu wurde das Wasser und Glycerin bei 70°C in einen Ansatztank mit Rührer und Wandheizung vorgelegt und die Gelatine in granulärer Form (ca. 1-3 mm Korn) unter Rühren eingebracht. Der Tank wurde evakuiert und bei reduziertem Druck (ca. 200 torr) während 90 min bis zu einer klaren blasenfreien Lösung gerührt.

Die Schmelze (heisse Lösung) wurde in eine beheizte Flachdüse gegossen und mittels Schwerkraft auf eine gekühlte Walze (18°C) zu einem Film gegossen, welcher sofort gummiartig erstarrte (Gel). Das Band (der Film) wurde von der Kühlwalze abgenommen und an der Luft getrocknet. Die so erhaltenen Filme wurden in Klimakammern konditioniert und mit Zugprüfmaschine (Instron 3345) bzw. Pendelschlagwerk (Zwick Typ B5102.202) vermessen.

Es wurden unter verschiedenen Konditionierungsbedingungen folgende Resultate erhalten:

| Konditionierungs-bedingungen | Schlagzähigkeit (kJ/m²) |
|---|---|
| 25°C, 50% RH | >560 |
| 30°C, 20% RH | 108 |

Es zeigte sich somit, dass bei den stärkehaltigen Massen und Gelatinemassen aus dem Stand der Technik (Vergleichsbeispiel 1 und 2) eine viele stärkere Erniedrigung der Schlagzähigkeit beobachtet wurde, wenn die Materialien einer höheren Temperatur bei niedrigerer Luftfeuchtigkeit ausgesetzt waren.

Die erfindungsgemässen Materialien eignen sich insbesondere als Hüllmaterialien bei der Herstellung von Formkörpern. Besonders bevorzugt können sie zur Herstellung von Weichkapseln mit Hilfe des Rotary Die-Verfahrens eingesetzt werden.

## Patentansprüche

1. Homogene, ungetrocknete, schmelzextrudierte thermoplastische Masse, enthaltend 30-60 Gew.-% Trockensubstanz an nativer oder chemisch modifizierter Stärke, höchstens 11 Gew.-% Trockensubstanz mindestens eines weiteren Biopolymers ausgewählt aus der Gruppe bestehend aus Carrageenan oder einem anderen Polysaccharid oder einem Protein, mindestens einen Weichmacher und maximal 20 Gew.-% zugesetztem Wasser.

2. Masse nach Anspruch 1, **dadurch gekennzeichnet, dass** die Masse 33-55 Gew.-% Trockensubstanz an nativer oder chemisch modifizierter Stärke und 3 bis 10,5 Gew.-% Trockensubstanz des weiteren Biopolymers enthält.

3. Masse nach Anspruch 1 oder 2, **dadurch gekennzeichnet, dass** der Weichmacher ausgewählt ist aus der Gruppe bestehend aus Glycerin, Sorbitol, Maltitol und hydrierten Stärkeabbauprodukten, oder Mischungen davon.

4. Masse nach einem der Ansprüche 1 bis 3, **dadurch gekennzeichnet, dass** der Weichmachergehalt der Masse 20 bis 45 Gew.-% Trockensubstanz beträgt.

5. Masse nach einem der Ansprüche 1 bis 4, **dadurch gekennzeichnet, dass** die Stärke Tapiokastärke und das Biopolymer iota-Carrageenan oder Sojaprotein oder Weizenprotein ist.

6. Masse nach einem der Ansprüche 1 bis 4, **dadurch gekennzeichnet, dass** die Stärke Kartoffelstärke und das Biopolymer Sojaprotein ist.

7. Masse nach einem der Ansprüche 1 bis 6, zusätzlich enthaltend mindestens einen Zusatzstoff ausgewählt aus der Gruppe bestehend aus Gleit- und Formtrennmitteln und Zuschlagstoffen.

8. Verfahren zur Herstellung einer homogenen, thermoplastischen Masse gemäss einem der Ansprüche 1 bis 7, umfassend die Schritte
a) Mischen von 30-60 Gew.-% Trockensubstanz an nativer oder chemisch modifizierter Stärke, höchstens 11 Gew.-% Trockensubstanz mindestens eines weiteren Biopolymers ausgewählt aus der Gruppe bestehend aus Carrageenan oder einem anderen Polysaccharid oder einem Protein, mindestens einen Weichmacher und maximal 20 Gew.-% zugesetztem Wasser
b) Dosieren der in Schritt a) erhaltenen Mischung in einen Extruder und Aufschmelzen im Extruder bei erhöhtem Druck von 10 bis 300 atm, erhöhter Temperatur von mindestens 40°C über Raumtemperatur und vorzugsweise einer Produkt-Temperatur von 60-150°C, erhöhter Scherleistung von 0.15 bis 0.67 kWh/kg und kurzer Verweildauer zwischen erstem Kontakt aller Komponenten nach Dosierung in den Extruder und dem Austritt der geschmolzenen homogenen Mischung aus dem Extruder von maximal 5 Minuten, bevorzugt 3 Minuten, noch bevorzugter 2 Minuten, wobei der Gesamtwassergehalt der Masse weniger als 20 Gew.-% der Gesamtmasse beträgt, unter Erhalt einer homogenen thermoplastischen Masse.

9. Verfahren nach Anspruch 8, **dadurch gekennzeichnet, dass** die Masse nach Verlassen des Extruders in ein Kühlmedium, vorzugsweise ein lebensmittelgerechte Kühlmedium, beispielsweise ein mittelkettiges Trigylcerid, eingeführt wird.

10. Formkörper, vorzugsweise Weichkapsel, umfassend eine Hülle aus einer homogenen thermoplastischen Masse nach einem der Ansprüche 1 bis 7.

11. Verfahren zur Herstellung eines Formkörpers, vorzugsweise einer Weichkapsel, umfassend die Schritte
a) Herstellung einer homogenen, thermoplastischen Masse gemäss einem der Ansprüche 8 oder 9,
b) Formung eines Formkörpers aus der in Schritt a) erhaltenen Masse in einem Formverfahren, vorzugsweise dem Rotary Die-Verfahren.

12. Verwendung einer homogenen thermoplastischen Masse nach einem der Ansprüche 1 bis 7 zur Herstellung von Formkörpern.

13. Verwendung nach Anspruch 12, **dadurch gekennzeichnet, dass** der Formkörper eine Weichkapsel ist.
